# EUROPEAN PATENT APPLICATION

(11) **EP 0 771 574 A1**
(43) Date of publication of application: **07.05.1997**
(21) Application number: 96307886.0
(22) Date of filing: 31.10.1996
(51) Int. Cl.: A61M 39/06

(54) **Gasket for catheter hemostasis valve**

(30) Priority: 01.11.1995 US 551509
(71) Applicant: Cordis Corporation, Miami Lakes Florida 33014 (US)
(72) Inventor: Davilla, Luis A., Terrace Cooper City, Florida 33330 (US); De La Mata, Carlo R., North Miami Beach, Florida 33160 (US)
(74) Representative: Mercer, Christopher Paul

(57) **Abstract**

A valve partition (36) used in a hemostasis valve of a catheter includes a body having first (38) and second (40) opposing surfaces and an opening (42) extending between the first and second surfaces. A portion of at least one of the first and second surfaces has a coarse topography (44) that reduces its surface area. The coarse topography includes peaks (46) and valleys (48).

## Description

The present invention relates to the field of catheters, and in particular, to gaskets for catheter hemostasis valves.

### BACKGROUND OF THE INVENTION

It is well known for catheter sheath introducers to have a hemostasis valve mounted in a housing on the end of a catheter. Examples of such devices are provided in U.S. Patents Nos.: 4,798,594 to Hillstead; 4,895,565 to Hillstead; 4,000,739 to Stevens; and 4,421,296 to Stevens. A catheter sheath introducer is used to facilitate the introduction of other catheters and guidewires into the vascular system of a patient, while minimizing injury to the patient at the access site. This is particularly used in situations where one or more catheters are inserted into and removed from the patient repeatedly, for example, in angioplasty. The presence of the catheter sheath introducer limits trauma to the body to only one catheter entering at the body access site. All other catheters and guidewires pass through the catheter sheath introducer, and thus are not traumatic to the body at the access site.

Catheter sheath introducers include a hemostasis valve with a slitted elastomeric gasket or partition carried in a housing. While the prior art discloses many different designs of such hemostasis valves, it is desirable to improve the performance of such valves with respect to their ability to seal against leakage of blood, as catheters and guidewires of varying diameters are passed through the valve. Also, it is important for lubricity, *i.e.*, friction encountered as one advances a catheter or a guidewire through the gasket of a catheter sheath introducer, to be as low as possible.

Attempts have been made to improve the lubricity of prior hemostasis valve gaskets, including adjusting the diameter and the thickness of such gaskets, as well as modifying the slits. Such attempts were oftentimes unsuccessful because any improved lubricity usually came at the expense of hemostasis.

### SUMMARY OF THE INVENTION

A gasket or valve partition constructed in accordance with the invention has high lubricity without compromising hemostasis. The valve partition is used in a hemostasis valve of a catheter, and includes a body having first and second opposing surfaces and an opening extending between the first and second surfaces. A periphery of the valve partition body is adapted to be secured within the hemostasis valve.

A contact portion of at least one of the first and second surfaces surrounds the opening at a location where the opening intersects the first and second surfaces. The contact portion has a coarse topography that reduces the surface area of the valve partition in contact with catheters or guide wires inserted into the catheter sheath introducer. The body typically has a circular disk-shape, and the opening is a slitted opening.

The coarse topography includes peaks and valleys that may have any shape. The peaks are preferably convex with respect to the body and protrude from both the first and second surfaces, with the valleys being disposed in the area between the peaks. Alternatively, the valleys may be recessed in the first and second surfaces and be concave with respect to the body, the peaks being disposed in the area between the valleys. The partition may also include convex peaks protruding from the body and concave valleys recessed in the body.

The coarse topography provides the hemostasis valve partition with a reduced contact surface area. That is, catheters and the like introduced into the catheter sheath introducer engage only the peaks or a portion thereof. The topography affords the valve partition with increased lubricity without compromising the hemostasis of the hemostasis valve. This is because the interior surfaces of the slits that contact the guidewires and catheters are not altered, but rather the surface area of the contact portion is reduced. Moreover, with respect to a valve partition having a conventional size, shape and slitted opening, catheters and guide wires having larger diameters than are typically used in such valve partitions can be inserted into the slitted opening of the present valve partition without adversely affecting hemostasis.

The foregoing and other features and advantages of the invention are illustrated in the accompanying drawings and are described in more detail in the specification and claims that follow.

### BRIEF DESCRIPTION OF THE DRAWINGS

Figure 1 is a cross-sectional view generally showing a valve partition constructed in accordance with the invention, disposed in a hemostasis valve of a catheter sheath introducer;
Figure 2 is a top view of the valve partition of the invention;
Figure 3 is a cross-sectional view of one embodiment of the valve partition taken along the plane indicated by lines 3-3 of Figure 2;
Figure 4 is a cross-sectional view of another embodiment of the valve partition taken along the plane indicated by lines 4-4 of Figure 2.

### DESCRIPTION OF PREFERRED EMBODIMENTS

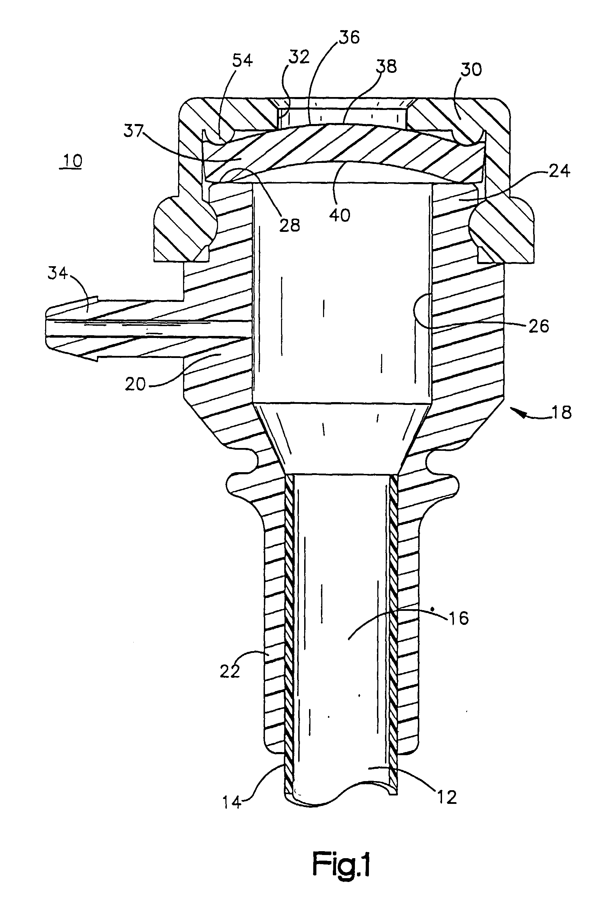

Turning now to the drawings, Figure 1 shows a catheter sheath introducer generally at 10. The catheter sheath introducer 10 includes a tubular catheter 12 having a distal end (not shown), a proximal end 14, and a lumen 16 extending therethrough. The catheter sheath introducer 10 includes a hemostasis valve 18 on one end thereof connected to the proximal end 14 of the catheter 12. The hemostasis valve 18 includes a housing 20 having a distal end 22, a proximal end 24 and a housing bore 26 extending therethrough. The distal end 22 of the housing 20 is attached by any suitable means known in the art to the proximal end 14 of the catheter 12. The housing bore 26 communicates with the lumen 16 of the catheter 12. The housing 20 has an end wall 28 at its proximal end 24. The housing 20 is closed at its proximal end 24 by an end cap 30 that is snap-fit and/or heat sealed thereto. The end cap 30 has a central aperture 32. The housing 20 includes a side port 34 typically found in catheter sheath introducers for introducing fluids such as heparin into the housing bore 26.

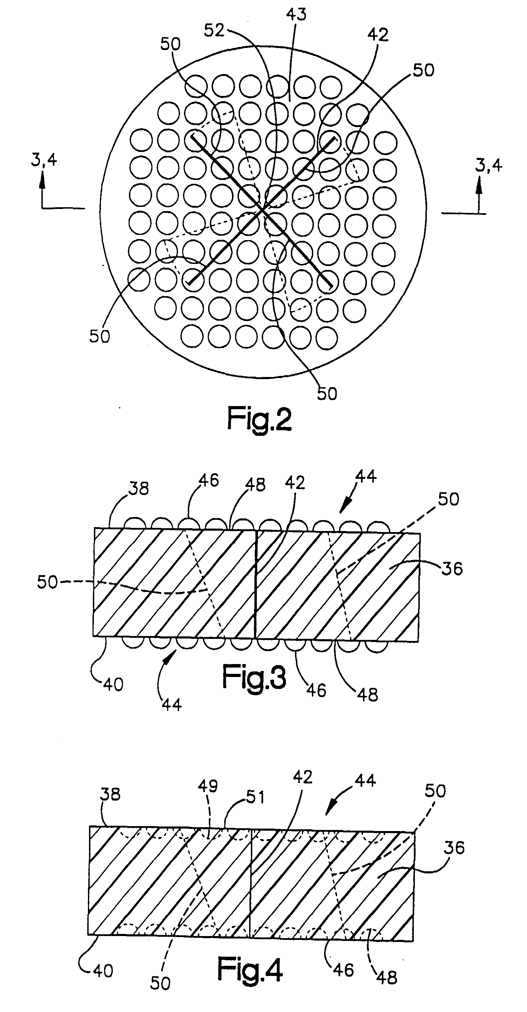

The hemostasis valve 18 includes a gasket or valve partition 36, shown generally in Figure 1. The valve partition 36 is preferably in the shape of a circular disk, having a body 37 with opposing first and second surfaces 38, 40, respectively. As shown in Figures 2-4, an opening 42 extends between the first and second surfaces 38, 40.

A contact portion 43 of at least one of the first and second surfaces 38, 40 is contacted by catheters, guidewires, and the like introduced through the opening 42. The contact portion 43 has a coarse topography 44 that reduces the surface area of the valve partition 36 in contact with the catheters or guide wires inserted into the catheter sheath introducer. It is preferable for the coarse topography 44 to include peaks 46 and valleys 48, and for these to be formed on the entirety of the first and second surfaces 38, 40. The peaks and valleys of the coarse topography 44 may be arranged in an ordered pattern as shown in Figure 2 or in an entirely random pattern.

As shown in Figure 3, the peaks 46 are preferably convex with respect to the body 37 and protrude from the first and second surfaces 38, 40, with the configuration of the valleys 48 being defined by the area between the peaks 46. Alternatively, as shown in Figure 4, valleys 49 may be recessed in the first and second surfaces 38, 40 and concave with respect to the body 37. The configuration of peaks 51 is defined by the area between the valleys 49.

The peaks and the valleys may have any geometric shape. However, from the standpoint of processing and effectiveness in improving lubricity, the peaks and valleys are preferably processed in a shape, such as a pyramid-like shape, that comes to a point. After such processing, the peaks 46 and the valleys 48 of the valve partition 36 may tend to resemble the rounded or hemispherical shape shown in Figure 3 or may be parabolic, due to trapped air in openings of the mold.

The opening 42 of the valve partition 36 is preferably slitted as in known designs, including a plurality of slits 50 (Figure 2) extending substantially radially from the center 52. The slits 50 are arranged in a helical manner as they extend through the partition 36 between the first surface 38 and the second surface 40, as shown by the dotted lines in Figures 2-4. The radial slits 50 circumferentially traverse the valve partition 36 by about 5° to 60° as they extend between the first surface 38 and the second surface 40.

The helical array of the slits 50 of the invention is similar to that disclosed in U.S. Patents Nos. 4,798,594 and 4,895,565 to Hillstead, which are incorporated herein by reference in their entirety. The valve partition 36 of the present invention employs three or more slits, with four slits being preferred. Although the specific helical array of the slits 50 shown in Figures 2-4 is preferred, any other configuration of slits known to those skilled in the art may be used.

The body 37 of the valve partition 36 is formed by a typical silastic molding process. In this process an electrode that is approximately the shape of the valve partition 36 is used to burn out a cavity in a mold material to form a reverse image of the partition in the resulting mold. The electrode provides the mold with the reverse image of the surface topography 44.

The valve partition 36 is then formed by adding elastomeric material to the mold and curing in the usual manner. The slits 50 are preferably formed in the valve partition 36 in the manner described in U.S. Patent No. 4,798,594, with a helical cutting blade.

Suitable surface finishes of the valve partition 36 are defined by a surface roughness average, which as referred to herein means the arithmetic average of the absolute values of measured profile height deviations taken within a sampling length and measured from a graphical centerline. The average surface roughness is determined graphically by measuring the height deviations normal to the centerline. The surface finish of the partition of the invention has an average surface roughness ranging from 0.8 µm to 6.3 µm, and more preferably, within the range of 1.6 µm to 2.2 µm.

The valve partition 36 is sealed around its periphery between the end wall 28 and the end cap 30. The end cap aperture 32 and the housing bore 26 communicate with the first and second surfaces 38, 40 of the valve partition 36, respectively.

The end cap 30 includes a compression ring 54 that abuts against the periphery of the valve partition 36. The compression ring 54 substantially surrounds the end cap aperture 32 and the portion of the housing bore 26 that is adjacent the partition 36. The compression ring 54 has a larger inner diameter than the diameter of the end cap aperture 32 and the housing bore 26. However, the outer diameter of the compression ring 54 is less than or equal to the diameter of the partition 36. This is because the ring 54 preferably engages the partition 36 along its periphery so as to press the peripheral portion of the partition 36 to a greater degree than the rest of the end cap 30 presses against the partition 36.

The effect of the inner diameter of the ring 54 being greater than the diameter of the housing bore 26 is to cause the partition 36 to bow outwardly as shown in Figure 1, and particularly to project outwardly to a degree into the end cap aperture 32. This outward bowing is facilitated by the space provided underneath the end cap 30 by the presence of the ring 54. The bowing is achieved primarily when the inner diameter of the ring 54 is greater than the diameter of the housing bore 26. The effect of such bowing enhances the sealing of the valve partition 36, particularly in the portion adjacent the second surface 40 of the partition 36. The extent of the bowing of the partition 36 may be adjusted by varying the dimensions of the ring 54 and its respective inner diameter compared with the inner diameter of the housing bore 26.

The partition 36 may be formed of any known composition used for hemostasis valve partitions. However, the valve partition 36 is preferably formed of silicone rubber, for example, silicone rubber which has an elongation to break of at least 900%, and typically no more than 1500%, as measured by the usual and conventional ASTM D412. Typical prior art slitted hemostasis valves have a maximum elongation of about 750% as measured in a similar manner. It has been found that the higher elongations used in the present invention help to provide improved sealing characteristics over partitions of lower elongation.

The elastomeric material preferably has a tensile strength of at least 11.5 megapascals up to a practical maximum of about 15 megapascals, as measured by ASTM D412. Typical prior art silicone partition valves have a tensile strength of about 10.9 megapascals, significantly below the tensile strength of the preferred elastomer materials used herein.

It is also preferred for the elastomeric materials used herein to have a Shore A durometer of about 20 to 50, although higher durometers may be used if desired. Silicone elastomer materials which are preferred for use herein are available from the Dow Corning Corporation of Midland, Michigan under the name Silastic Q7-47-20, 35, or 50 medical grade ETR elastomer. The later two-digit numbers refer to the Shore A durometers of the material. A preferred tear strength of the elastomers used herein is at least 150 pounds/inch as determined by ASTM D624, Die B.

In accordance with the invention, the valve partition 36 is preferably made of an elastomer such as the ones described above, and also contains an effective amount of a lubricity enhancing additive which is a finely divided material such as bismuth oxychloride, polytetrafluorethylene, titanium dioxide, graphite, polyethylene wax, polyvinylpyrrolidone, and combinations thereof. The lubricity enhancing additives are typically milled into the uncured elastomer, so as to be substantially uniformly distributed throughout the elastomer material. The graphite is used in a concentration of about 5 to 15 weight percent. Each of the other lubricity enhancing additives may be present in a total amount of about 5 to 20 weight percent in the elastomer formulation.

Each of the additives is preferably present in a concentration of about 7 to 13 weight percent. By way of specific embodiment, formulations of the above described silicone elastomer have been prepared, wherein each formulation contained 10 weight percent of one of the following: bismuth oxychloride, PTFE, titanium dioxide, polyethylene wax, and polyvinylpyrrolidone. Alternatively, a similar formulation of such a silicone elastomer has been prepared containing 7.5 weight percent of graphite. The specific Shore A durometers of the silicone rubber silastic ETR Q7-47 formulations tested were 20, 35, and 50.

The coarse topography 44 of the present invention reduces the friction encountered when a catheter or guidewire is advanced through the slitted opening 42, while not adversely affecting hemostasis. In addition, by using the coarse topography on both the first and second surfaces 38, 40 of the valve partition, lubricity is enhanced when the catheters and guidewires are inserted as well as when they are removed.

The lubricity enhancing additives of the invention further reduce the friction encountered when a catheter or guidewire is advanced through the slitted opening 42. Also, the lubricity enhancing additives prevent leaflets of elastomer material defined by the slits 50 in the partition 36 from sticking to each other with high force. It is possible for the slits 50 cut in partitions 36 made of the unmodified elastomer to actually reseal because of the highly tacky characteristic of most silicone elastomers of high elongation, especially when the preferred elastomers of high elongation are used as the valve partition 36. The presence of one or more of the additives of the invention may reduce or eliminate this phenomenon, while also reducing frictional forces as a catheter or guidewire is advanced through the valve partition 36.

Although the invention has been described in its preferred form with a certain degree of particularity, it will be understood that the present disclosure of the preferred embodiments has been made only by way of example, and that various changes may be resorted to without departing from the true spirit and scope of the invention as hereinafter claimed.

## Claims

1. A valve partition for a hemostasis valve comprising
a body having first and second opposing surfaces and an opening extending between said first and second surfaces,
at least a portion of at least one of said first and second surfaces adjacent said opening having a coarse topography.

2. The valve partition of claim 1 wherein said topography comprises peaks and valleys.

3. The valve partition of claim 2 wherein said peaks have a convex shape with respect to said body.

4. The valve partition of claim 2 or claim 3 wherein said valleys have a concave shape with respect to said body.

5. The valve partition of any one of claims 2 to 4 wherein said peaks protrude from said first and second surfaces.

6. The valve partition of any one of claims 2 to 5 wherein said valleys are recessed in said first and second surfaces.

7. The valve partition of any one of claims 2 to 6 wherein said peaks and said valleys form an average surface roughness with respect to said body ranging from 0.8 µm to 6.3 µm.

8. The valve partition of claim 7 wherein said peaks and said valleys form an average surface roughness with respect to said body ranging from 1.6 µm to 2.2 µm.

9. The valve partition of any one of claims 1 to 8 wherein said body has a circular disk-shape.

10. The valve partition of any one of claims 1 to 9 wherein said opening is a slitted opening.
